# EUROPEAN PATENT SPECIFICATION

(11) **EP 4 618 917 B1**
(45) Date of publication and mention of the grant of the patent: **15.04.2026**
(21) Application number: 23817929.5
(22) Date of filing: 13.11.2023
(51) Int. Cl.: A61F 5/443, A61F 5/445, A61F 5/448

(54) **OSTOMY BARRIER RELEASE LINER SYSTEM**
TRENNSCHICHTSYSTEM FÜR STOMABARRIERE
SYSTÈME DE DOUBLURE DÉTACHABLE DE BARRIÈRE DE STOMIE

(30) Priority: 14.11.2022 US 202263425015 P
(43) Date of publication of application: 24.09.2025
(73) Proprietor: Hollister Incorporated, Libertyville, IL 60048 (US)
(72) Inventor: JOCKEL, Mark, W., Libertyville, IL 60048 (US)
(74) Representative: FRKelly
(86) International application number: PCT/US2023/079471
(87) International publication number: WO 2024/107622

(56) References cited:
- GB-A- 2 553 096
- JP-A- 2016 538 897
- US-A1- 2017 007 439
- US-A1- 2019 125 570

## Description

### BACKGROUND

This disclosure is related to an ostomy barrier. More particularly, the present disclosure pertains to an ostomy barrier release liner system.

Ostomy pouches for collecting bodily waste are used by individuals who have had surgery such as a colostomy, ileostomy, or urostomy. An ostomy pouch may be attached to a user via an ostomy barrier, which is configured to seal against peristomal skin surfaces and protect the peristomal surfaces from exposure to stomal effluent. However, the topography of stomas and peristomal surfaces surrounding stomas varies among patients, and sealing an ostomy appliance against such different peristomal surfaces and stomas remains an area for further improvement. For example, an improper fit of the ostomy barrier to the skin may leave gaps or channels that may cause premature leakage.

A person applying an ostomy may find that aligning the ostomy barrier around the stoma and preparing the peristomal skin before securing the barrier to the skin may provide a better fit that prevents premature leakage. However, ostomy barriers require a liner to be removed before aligning the barrier around the stoma and securing the barrier to the skin. It has been found that, at times, even the most careful individuals may misalign the barrier and not properly prepare the peristomal skin surface. This may occur, for example, unless the barrier is partially applied while aligning the barrier to the stoma.

US2017007439A1 discloses an accessory and/or manufacturing stage addition to current ostomy devices which is capable of remediating or preventing various causes for concerns for the myriad populations of patients who rely on such devices.

Accordingly, it is desirable to provide an improved ostomy barrier appliance that allows alignment of the barrier around the stoma and preparation of the peristomal skin. In this description, 1 inch will be considered to be equal to 2,54 cm.

### BRIEF SUMMARY

The invention concerns an adhesive device according to claim 1, as illustrated in the following embodiments.

In an embodiment, the adhesive device may be an ostomy barrier assembly for attaching an ostomy appliance to a peristomal skin surrounding a stoma. The ostomy barrier assembly may include a skin barrier comprising the adhesive, an inlet opening defined in the skin barrier for receiving the stoma, and the two-piece release liner system attached to the skin barrier. The two-piece release liner system may be configured to allow a user to expose the adhesive to the peristomal skin while the skin barrier is aligned with and surrounds the stoma.

In an embodiment, the two-piece release liner system may be configured such that the first release liner covers about a half of the adhesive and the second release liner covers the other half of the adhesive. In such an embodiment, a split between the first and second release liners may be defined near a center of the adhesive. In some embodiments, the two-piece release liner system may include a center opening aligned with the inlet opening.

In an embodiment, at least one of the first and second release liners may be configured to extend past a periphery of the adhesive. In some embodiments, the second release liner may overlap the first release liner near a center of the adhesive.

In an embodiment, the first release liner may include the first grasping portion, a first mid-portion, and the first contact portion, wherein the first grasping portion folds onto the first mid-portion at a first crease and the first mid-portion folds onto the first contact portion at a second crease. The first release liner may be configured such that the first grasping portion extends beyond a periphery of the adhesive device when the first grasping portion is unfolded away from the first mid-portion. The first release liner may be configured to be removed from the adhesive by a pulling motion of a user, wherein the at least one finger hole is configured to receive user's finger to facilitate grasping of the first grasping portion and pulling away the first contact portion towards an outer periphery of the adhesive device.

In an embodiment, the second release liner may be configured as a mirror image of the first release liner and may include a second grasping portion, a second mid-portion, and a second contact portion. In such an embodiment, the second release liner may be configured such that the second grasping portion folds onto the second mid-portion at a third crease and the second mid-portion folds onto the second contact portion at a fourth crease. The second release liner may be configured such that the second grasping portion extends beyond a periphery of the adhesive device when the second grasping portion is unfolded away from the second mid-portion. The second grasping portion may include at least one finger hole configured to receive user's finger to facilitate grasping of the second grasping portion and pulling away the second contact portion towards an outer periphery of the adhesive device to remove the second release liner from the adhesive by a pulling motion of a user.

In an embodiment, the two-piece release liner system may include a strip of tape configured to hold the first and second grasping portions together.

In an embodiment, an adhesive facing surface of the first contact portion and an adhesive facing surface of the second contact portion may be coated with a release coating configured for releasable attachment of the first and second contact portions to the adhesive. In some embodiments, at least a portion of the first and second grasping portions and/or at least a portion of the first and second mid-portions may be configured as a writeable surface to allow a user to write on the writeable surface using a marker or a pen.

In an embodiment, the first and second release liners may include a reinforced material for preventing tearing during removal.

In an embodiment, the skin barrier may be a convex barrier or a concave skin barrier.

In an embodiment, the adhesive device may be a medical adhesive device for attaching a suction tube, a drain tube, a vertical tube, a feeding tube, a horizontal tube, or an oral endotracheal tube to a user.

In an embodiment, the adhesive device may include a clamping mechanism configured to facilitate grabbing and removing the first and second release liners.

### BRIEF DESCRIPTION OF THE DRAWINGS

The benefits and advantages of the present embodiments will become more readily apparent to those of ordinary skill in the relevant art after reviewing the following detailed description and accompanying drawings, wherein:
FIG. 1 is a distal side view of an ostomy barrier appliance according to an embodiment.
FIG. 2 is a schematic cross-sectional view of the ostomy barrier appliance of FIG. 1 with a two-piece split release liner partially open.
FIG. 3 is a front side view of one of the two-piece release liners unfolded.
FIG. 4 is a body side view of the ostomy barrier appliance of FIG. 1.
FIG. 5 is a distal side view of the ostomy barrier appliance of FIG. 1 with the two-piece split release liner partially open.
FIG. 6 is a body side view of the ostomy barrier appliance of FIG. 1 with one of the two-piece split release liners partially open.
FIG. 7A is a distal side view of the ostomy barrier appliance of FIG. 1 applied to a stoma and with the two-piece split release liner partially open.
FIG. 7B is a distal side view of the ostomy barrier appliance of FIG. 1 applied to the stoma and with one of the two-piece split release liners being removed.
FIG. 7C is a distal side view of the ostomy barrier appliance of FIG. 1 applied to the stoma and with the second of the two-piece split release liners being removed.
FIG. 7D is a distal side view of the ostomy barrier appliance of FIG. 1 applied to the stoma and with the two-piece split release liners fully removed and the barrier positioned on the stoma and secured to a patient.
FIG. 8 is a body side view of an ostomy barrier appliance including a tape for holding two release liners together according to an embodiment.

### DETAILED DESCRIPTION

The words "a" or "an" are to be taken to include both the singular and the plural. Conversely, any reference to plural items shall, where appropriate, include the singular.

The present disclosure provides a two-piece split release liner system. The release liners can be part of an ostomy barrier appliance. The release liners can be applied to an ostomy barrier of a one-piece pouch system or a faceplate for a two-piece pouch system. The release liners can be used to align the barrier around a stoma and prepare a peristomal skin before removing each of the release liners and securing the barrier to the skin of a user. The two-piece release liner system may also be used for concave skin barriers or other skin contact adhesive devices, where it is beneficial to place the adhesive device against user's skin and check the position and sizing of the device before exposing an adhesive to the user's skin. For example, the two-piece release liner system may be used to protect an adhesive surface of medical devices of various shapes, such as suction tube attachment devices, drain tube attachment devices, vertical tube attachment devices, feeding tube attachment devices, horizontal tube attachment devices, and oral endotracheal tube fasteners, which are available from Hollister Incorporated, the Applicant of the present disclosure.

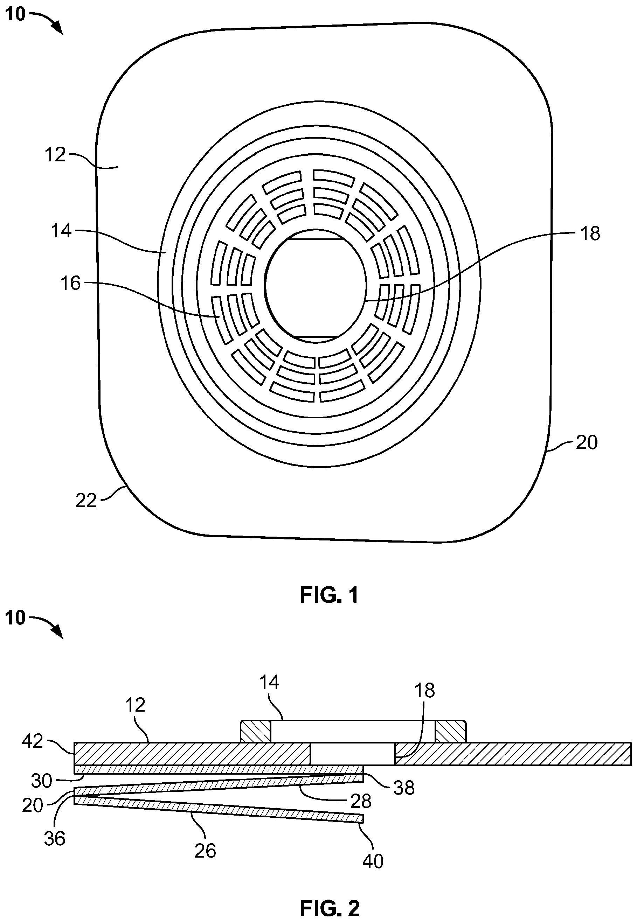

Referring now to the figures, FIGS. 1-7D show an ostomy barrier appliance 10 according to an embodiment. The illustrated appliance 10 is a two-piece appliance having a separate coupling member (not shown) that allows for attaching a pouch to the appliance 10.

FIG. 1 illustrates a distal view of an ostomy barrier appliance 10. The ostomy barrier appliance 10 can generally include a tape 12, a first portion 14 of a coupling for securing a pouch to a barrier, a skin barrier 16, an inlet opening 18 for receiving a stoma, a first release liner 20, and a second release liner 22.

The tape 12 may include an adhesive layer and a backing layer. The adhesive layer of the tape 12 can be formed from a suitable medical adhesive, such as an acrylic adhesive. The backing layer can be formed from a suitable material, such as a nonwoven material or a thin polymeric film. In another embodiment, the tape 12 can comprise a hydrocolloid adhesive and a film backing layer.

In some embodiments, the skin barrier 16 can include a backing layer laminated on the pouch-side surface of the skin barrier 16. The backing layer can be formed from a suitable heat sealable polymeric material, such that the backing layer can be heat sealed to the tape 12. The skin barrier 16 can be formed from a suitable skin-contact adhesive, such as hydrocolloid adhesives.

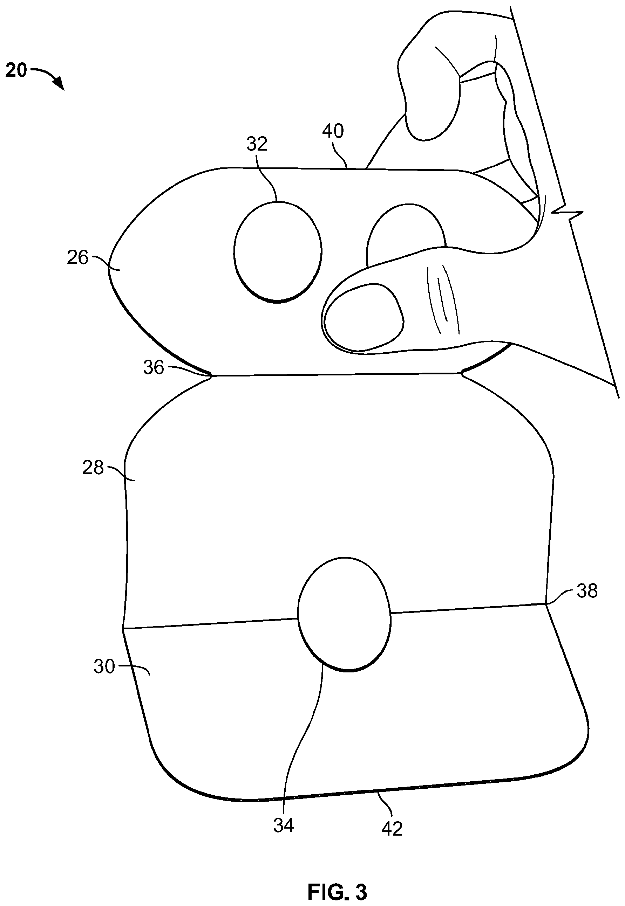

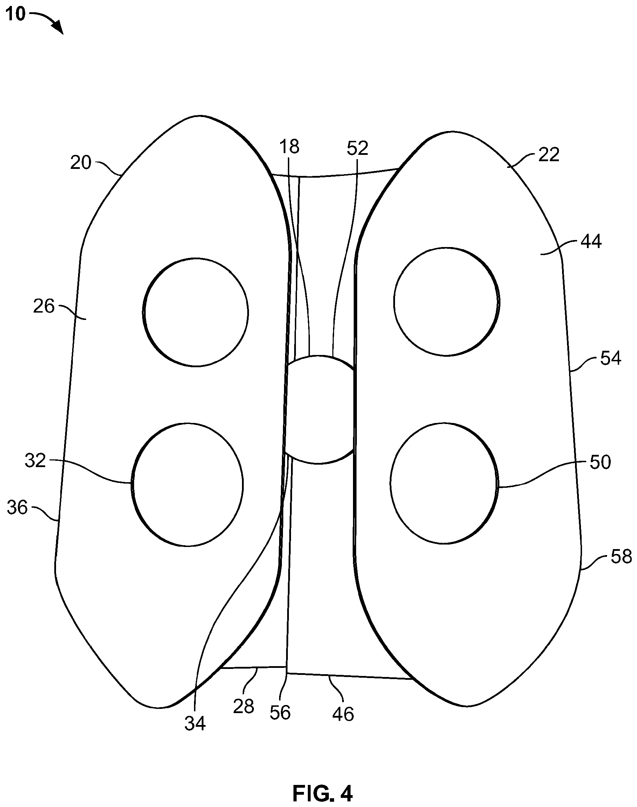

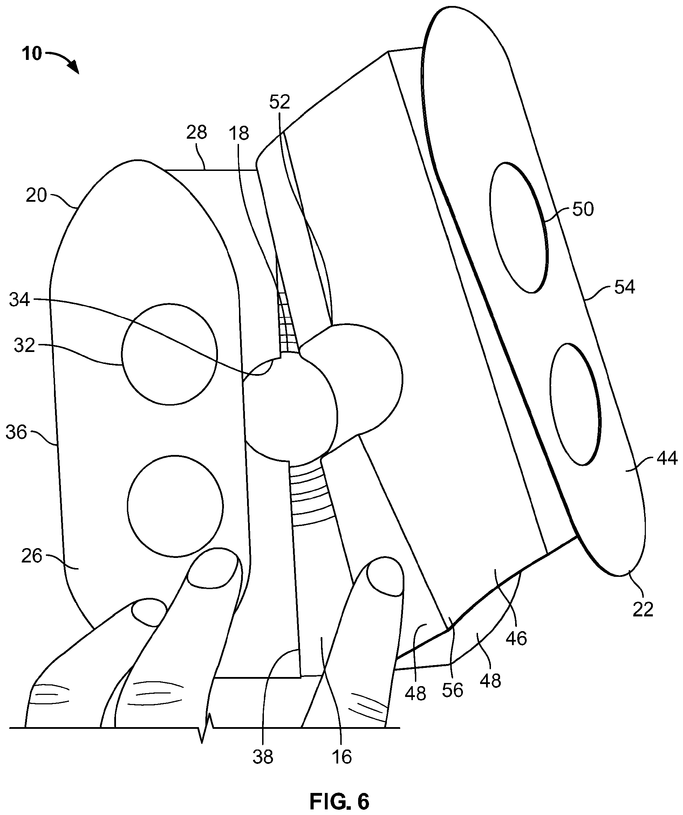

FIG. 2 illustrates a schematic cross-sectional view of the ostomy barrier appliance 10 with the first release liner 20 partially open and without the second release liner 22. FIG. 3 illustrates a front side view of the first release liner 20 unfolded. FIGS. 4 and 6 illustrate a body side view of the ostomy barrier appliance 10. The appliance 10 can include a two-piece split release liner comprising the first release liner 20 and the second release liner 22 attached to the body side of the tape 12 and skin barrier 16. The first release liner 20 can include a wing grasping portion 26, a mid-portion 28, a contact portion 30, a first crease 36, a second crease 38, a first end 40, and a second end 42 (FIG. 3). The first release liner 20 can further include one or more finger holes 32 and an opening 34 near the inlet opening 18. The first release liner 20 can be folded onto itself to form a z-like structure (FIG. 2). The wing grasping portion 26 can fold onto mid-portion 28 at the first crease 36 and the mid-portion 28 can fold onto the contact portion 30 at the second crease 38. The second end 42 can be located near an outer edge of tape 12, while the opening 34 can align with the inlet opening 18. The contact portion 30 may be configured to releaseably attach to the ostomy barrier appliance 10 and removed therefrom when pulled.

The second release liner 22 can be a mirror image of the first release liner 20 with a wing grasping portion 44, a mid-portion 46, a contact portion 48, a first crease 54, a second crease 56, a first end 58, and a second end 60 (FIGS. 4-6 and 7C). The second release liner 22 can also include one or more finger holes 50 and an opening 52 near inlet opening 18. The second release liner 22 can similarly be folded into a z-like structure as the first release liner 20.

The opening 34, 52 can be located between the mid-portion 28, 46 and the contact portion 30, 48 so that when the mid-portion 28, 46 and the contact portion 30, 48 are folded onto each other, the opening 34, 52 can align with the inlet opening 18. The release liners 20, 22 can be removed from the ostomy barrier appliance 10 by unfolding the wing grasping portion 26, 44 from the mid-portion 28, 46 and pulling on the wing grasping portion 26, 44 away from the ostomy barrier appliance 10. When the wing grasping portion 26, 44 is pulled, the contact portion 30, 48 may be pulled away from the ostomy barrier appliance 10 starting near or at the second crease 38, 56 towards the edge of the tape 12 thereby removing the release liners 20, 22 from the ostomy barrier appliance 10.

In an embodiment, the mid-portion 46 can overlap the mid-portion 28 (FIG. 4). This can allow for the adhesive to be fully covered by the release liners 20, 22 without a gap between them.

In an embodiment, the first and second release liners 20, 22 may be configured such that only adhesive facing surfaces of the contact portions 30, 48 are provided with a release coating. For example, the first and second release liners 20, 22 may be zone coated to apply a release coating, such as silicone release coating, only on the surfaces of the contact portions 30, 48 that are in contact with an adhesive. In such an embodiment, instructions and/or information related to the ostomy barrier appliance 10, for example, a cut-to-fit guide, may be printed on user facing surfaces of the first and second release liners 20, 22. The non-release coated surfaces of the first and second release liners 20, 22 may also be configured to allow a user to write with a marker or pen. In some embodiments, one or more surfaces of the first and second release liners 20, 22 may be coated with a writeable coating to provide writeable surfaces for users.

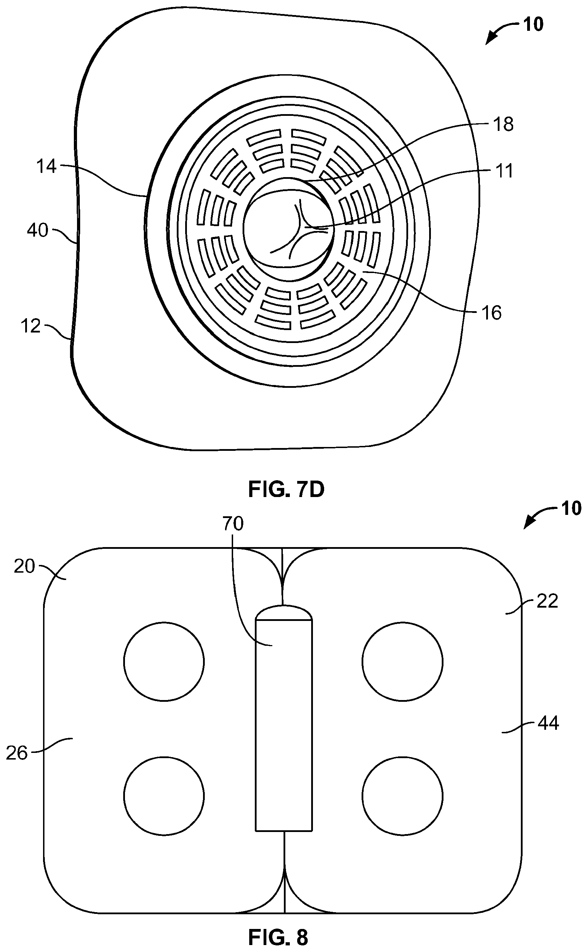

In an embodiment, a strip of tape 70 may be provided to hold the wing grasping portions 26, 44 together as shown in FIG. 8, so that the wing grasping portions 26, 44 may not be unfolded during storage or before application of the ostomy barrier appliance 10. The strip of tape 70 may be provided with an adhesive configured to releaseably attach to the wing grasping portions 26, 44 to hold them in place and easily removed by a user. The strip of tape 70 may have a user facing surface having a different color or a constrasting color to the release liners 20, 22 to facilitate removal of the strip of tape 70 by a user. In some embodiments, the user facing surface of the strip of tape 70 may include instructions and/or information printed thereon.

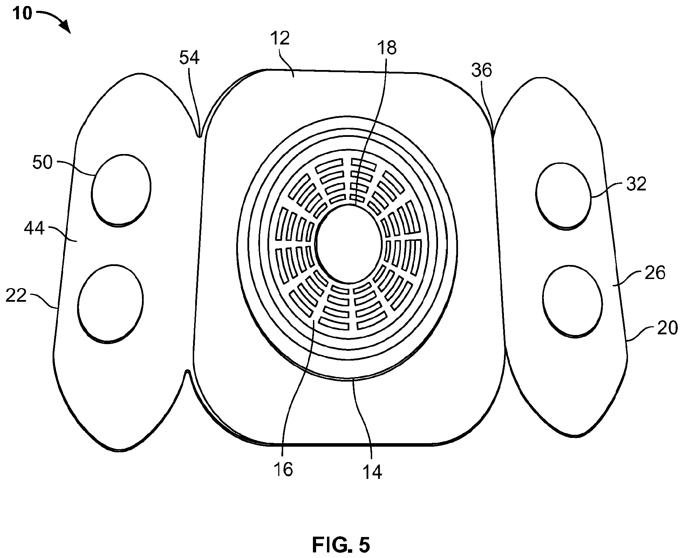

FIG. 5 illustrates a distal side view of the ostomy barrier appliance 10 with wing grasping portion 26, 44 unfolded. The release liners 20, 22 can be partially unfolded at the first crease 36, 54 to have the wing grasping portion 26, 44 extend past the edges of the tape 12.

FIG. 6 illustrates a body side view of the ostomy barrier appliance 10 with the second release liner 22 partially unfolded and pulled away proximate the second crease 56 to expose the skin barrier 16.

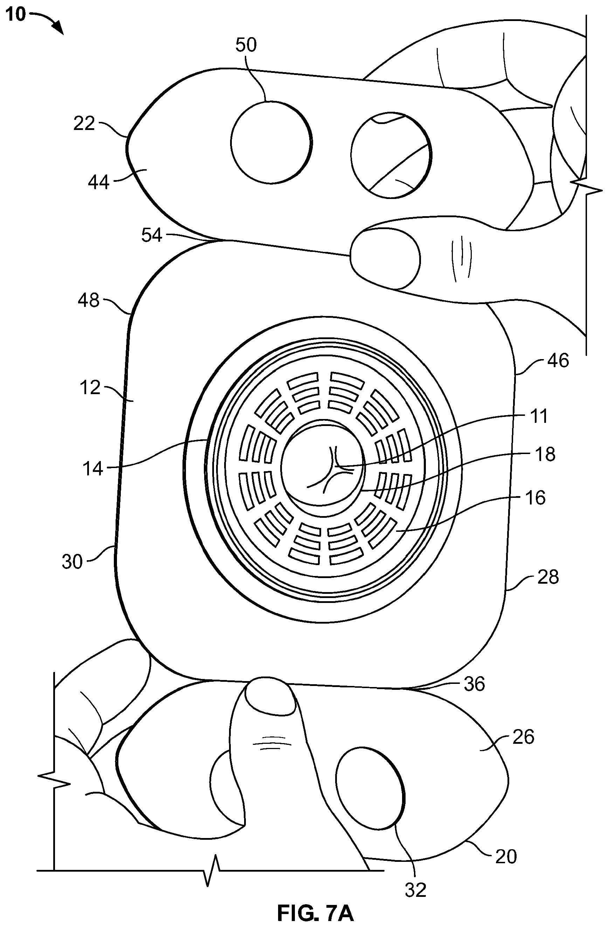

FIGS. 7A-7D illustrate a distal side view of the ostomy barrier appliance 10 as the release liners 20, 22 are being unfolded and removed from the ostomy barrier appliance 10 as it is applied around the stoma 11.

FIG. 7A shows the ostomy barrier appliance 10 with a skin barrier 16 surrounding a stoma 11 and the release liners 20, 22 being unfolded at first creases 36, 54 to expose the one or more finger holes 32, 50. The ostomy barrier appliance 10 can then be adjusted around the stoma 11 and smoothed out on the peristomal skin to facilitate attachment of the ostomy barrier appliance 10 to the user. The one or more finger holes 32, 50 can be used to grasp the wing grasping portion 26, 44 and pull on the release liners 20, 22 away from the inlet opening 18. Pulling on the release liners 20, 22 causes the second creases 38, 56 to be pulled away from the inlet opening 18 and expose the skin barrier 16 and the tape adhesive for attachment to the user's skin.

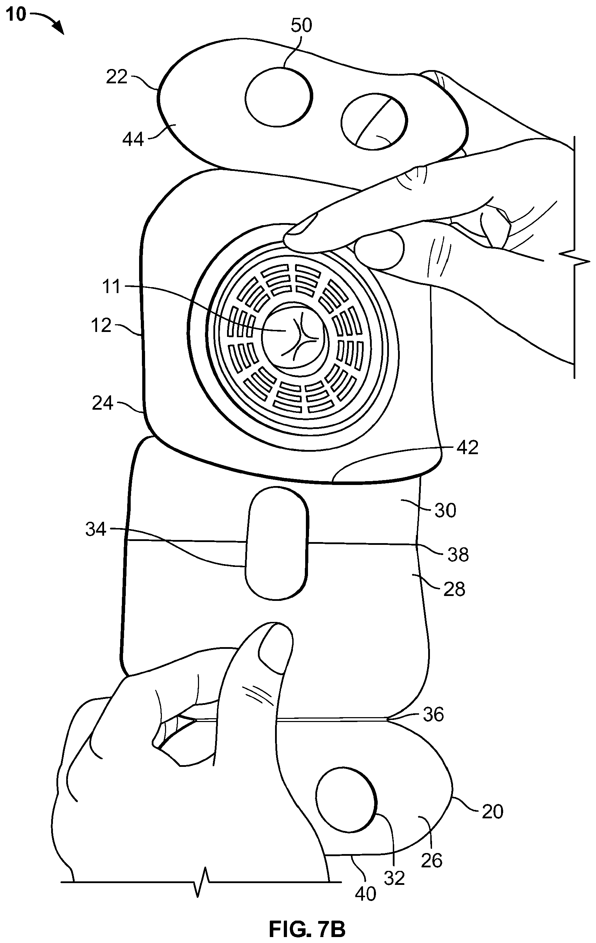

FIG. 7B shows the first release liner 20 being removed from the ostomy barrier 10 by pulling on the first release liner 20 away from the inlet opening 18 and towards the first end 40. The user, while pulling the first release liner 20, can adjust and/or smooth out the skin barrier 16 to facilitate attachment of the ostomy barrier appliance 10 to the peristomal skin. For example, the user may remove any air pockets or bumps in the skin barrier 10 while attaching the ostomy barrier appliance 10 to the peristomal skin.

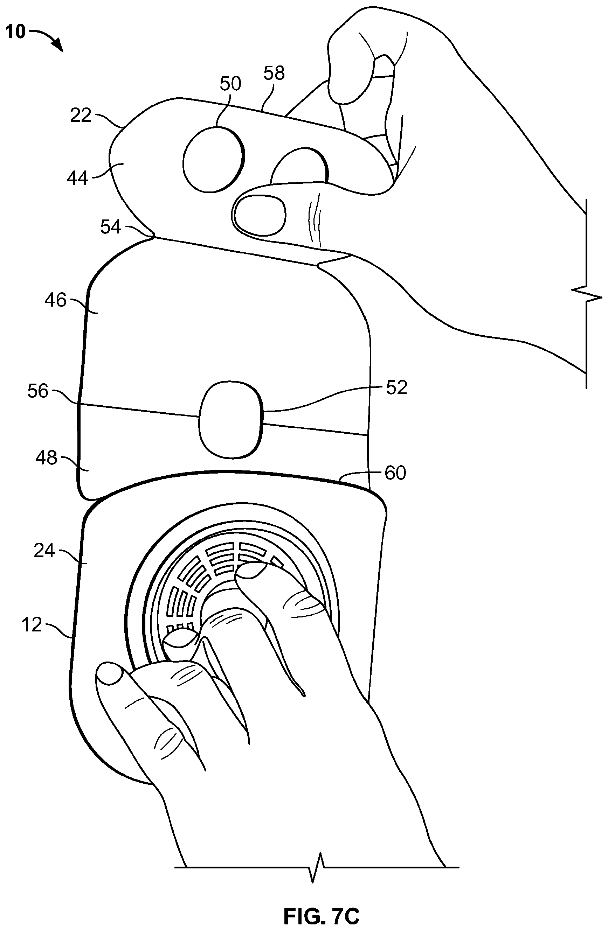

FIG. 7C shows the first release liner 20 fully removed and the second release liner 22 being removed.

FIG. 7D shows the release liners 20, 22 fully removed and the ostomy barrier appliance 10 attached to a user's skin (not shown) and surrounding the stoma 11.

The split release liner system allows for sequential removal of two release liners after the ostomy barrier appliance 10 is placed against the peristomal skin. This provides a user an opportunity to correct any errors in placement and smooth out of the ostomy barrier appliance 10 to ensure substantially complete contact between the peristomal skin and the ostomy barrier appliance 10, thereby minimizing a risk of a premature leakage. The split release liner system may minimize air pockets and gaps and may provide improved adhesion between the ostomy barrier applaince 10 and the peristomal skin.

The release liner 20, 22 may be configured to extend sufficiently past the peripheral edges of the ostomy barrier appliacne 10 when unfolded and configured to allow a user to check for alignment with the stoma, press the ostomy barrier appliance 10 to user's skin, smooth out the peristomal skin to ensure contact with the ostomy barrier appliance 10, and then pull off the release liners 20, 22.

In an embodiment, the release liner 20, 22 can have a wing grasping portion 26, 44 that extends past the edges of the ostomy barrier 10 when unfolded. In an embodiment, the wing grasping portion 26, 44 may extend two inches past the edges of the ostomy barrier 10 when unfolded. In other embodiments, the wing grasping portion 26, 44 may extend based on the size of the ostomy barrier being used. The wing grasping portion 26, 44 can include finger holes 32, 50 to allow a user, with limited dexterity, to place one or two fingers through the release liner to pull the release liner off the barrier.

In an embodiment the release liner 20, 22 can have a reinforcing element to keep the release liner from tearing during removal and provide more control to the user.

In an embodiment, the ostomy barrier 10 and/or skin barrier 16 can be flat or convex.

In an embodiment, release liner 20, 22 can be coated so that its coefficient of friction to the peristomal skin is low. The low friction coefficient will aid in moving the ostomy barrier, with release liners still on, to the right position when it is pressed against the skin. Furthermore, the coating can also allow the user to write on the back of the release liner with a pen or marker.

In an embodiment, the ostomy barrier assembly may further include an accessory that may also be used to aid in removing the release liner. The accessory may have a clamping mechanism to grab the release liner and provide a more comfortable or easier grip solution. The accessory may also allow for shorter lengths of release liners than without the accessory. The accessory may have a method to fasten to the release liner and then be connected to a length of material before ending with a gripping mechanism.

In an embodiment, the second release liner 22, which extends over the first release liner 20, can be folded over the second release liner 20 to allow it to be more easily manufactured and take up less space when packed. The second release liner 22 can be folded back onto itself and be ready to use when needed.

In an embodiment, each of the first and second release liners 20, 22 may be configured to be the same size as the ostomy barrier 10. For example, for a 4inch x 4inch ostomy barrier 10, each of the first and second release liner 20, 22 may be configured to have the substantially same size to the ostomy barrier 10, 4inch x 4inch. In such an embodiment, each of the first and second release liner 20, 22 may be folded in half for attachment to the ostomy barrier 10, wherein wing grasping portions may be provided with zig-zag cuts and configured to expand beyond an outer periphery of the ostomy barrier 10 to function similar to the foregoing embodiments of the release liners 20, 22.

The release liner can additionally minimize the formation of air pockets and other discontinuities between the adhesive and the surface to which it is being applied. The user can not only ensure better alignment, but a more continuous application of the barrier to the stoma and surrounding tissue.

## Claims

1. An adhesive device for attachment to skin comprising:
an adhesive for attachment to skin; and
a two-piece release liner system (20, 22) for protecting the adhesive,
wherein the two-piece release liner system includes a first release liner (20) and a second release liner (22), wherein the first release liner includes a first contact portion (30) configured to be attached to the adhesive and a first grasping portion (26), **characterized in that** of the first grasping portion including at least one finger hole (32) configured to facilitate removal of the first release liner by a user.

2. The adhesive device of claim 1, wherein the adhesive device is an ostomy barrier assembly (10) for attaching an ostomy appliance to a peristomal skin surrounding a stoma comprising:
a skin barrier (16) comprising the adhesive;
an inlet opening (18) defined in the skin barrier for receiving the stoma; and
the two-piece release liner system (20, 22) attached to the skin barrier;
wherein the two-piece release liner system is configured to allow a user to expose the adhesive to the peristomal skin while the skin barrier is aligned with and surrounds the stoma.

3. The adhesive device of claim 1 or 2, wherein the two-piece release liner system (20, 22) is configured such that the first release liner (20) covers about a half of the adhesive and the second release liner (22) covers the other half of the adhesive, wherein a split between the first and second release liners is defined near a center of the adhesive.

4. The adhesive device of claim 2 or 3, wherein the two-piece release liner system (20, 22) includes a center opening (34, 52) aligned with the inlet opening.

5. The adhesive device of any one of claims 1-4, wherein at least one of the first and second release liners (20, 22) is configured to extend past a periphery of the adhesive and/or wherein the second release liner (22) overlaps the first release liner (20) near a center of the adhesive.

6. The adhesive device of any one of claims 1-5, wherein the first release liner (20) includes the first grasping portion (26), a first mid-portion (28), and the first contact portion (30), wherein the first grasping portion folds onto the first mid-portion at a first crease (36) and the first mid-portion folds onto the first contact portion at a second crease (38), wherein the first release liner is configured such that the first grasping portion extends beyond a periphery of the adhesive device when the first grasping portion is unfolded away from the first mid-portion.

7. The adhesive device of claim 6, wherein the first release liner (20) is configured to be removed from the adhesive by a pulling motion of a user, wherein the at least one finger hole (32) is configured to receive user's finger to facilitate grasping of the first grasping portion (26) and pulling away the first contact portion (30) towards an outer periphery of the adhesive device.

8. The adhesive device of claim 6 or 7, wherein the second release liner (22) is configured as a mirror image of the first release liner (20) and includes a second grasping portion (44), a second mid-portion (46), and a second contact portion (48), wherein the second grasping portion folds onto the second mid-portion at a third crease (54) and the second mid-portion folds onto the second contact portion at a fourth crease (56), wherein the second release liner is configured such that the second grasping portion (44) extends beyond a periphery of the adhesive device when the second grasping portion is unfolded away from the second mid-portion.

9. The adhesive device of claim 8, wherein the second release liner (22) is configured to be removed from the adhesive by a pulling motion of a user, wherein the second grasping portion (44) includes at least one finger hole (50) configured to receive user's finger to facilitate grasping of the second grasping portion and pulling away the second contact portion (48) towards an outer periphery of the adhesive device.

10. The adhesive device of any one of claims 8-9, wherein the two-piece release liner system (20, 22) includes a strip of tape (70) configured to hold the first and second grasping portions (26, 44) together.

11. The adhesive device of any one of claims 6-10, wherein an adhesive facing surface of the first contact portion (30) and an adhesive facing surface of the second contact portion (48) are coated with a release coating configured for releasable attachment of the first and second contact portions (30, 48) to the adhesive.

12. The adhesive device of any one of claims 6-11, wherein at least a portion of the first and second grasping portions (26, 44) and/or at least a portion of the first and second mid-portions (28, 46) is configured as a writeable surface to allow a user to write on the writeable surface using a marker or a pen.

13. The adhesive device of any one of claims 1-12, wherein the first and second release liners (20, 22) comprise a reinforced material for preventing tearing during removal, and/or the adhesive device further comprising a clamping mechanism configured to facilitate grabbing and removing the first and second release liners (20, 22).

14. The adhesive device of any one of claims 2-13, wherein the skin barrier (16) is a convex barrier or a concave skin barrier.

15. The adhesive device of claim 1, wherein the adhesive device is a medical adhesive device for attaching a suction tube, a drain tube, a vertical tube, a feeding tube, a horizontal tube, or an oral endotracheal tube to a user.

## Patentansprüche

1. Klebevorrichtung zur Befestigung an der Haut, umfassend:
einen Klebstoff zur Befestigung auf der Haut; und
ein zweiteiliges Trennschichtsystem (20, 22) zum Schutz des Klebstoffs,
wobei das zweiteilige Trennschichtsystem eine erste Trennschicht (20) und eine zweite Trennschicht (22) beinhaltet, wobei die erste Trennschicht einen ersten Kontaktabschnitt (30), der dazu konfiguriert ist, am Klebstoff befestigt zu werden, und einen ersten Greifabschnitt (26) beinhaltet, **dadurch gekennzeichnet, dass** der erste Greifabschnitt mindestens ein Fingerloch (32) beinhaltet, das dazu konfiguriert ist, ein Entfernen der ersten Trennschicht durch einen Benutzer zu erleichtern.

2. Klebevorrichtung nach Anspruch 1, wobei die Klebevorrichtung eine Stomabarrierebaugruppe (10) zum Befestigen einer Stomaeinrichtung an einer ein Stoma umgebenden peristomalen Haut ist, umfassend:
eine Hautbarriere (16), die den Klebstoff umfasst;
eine in der Hautbarriere definierte Einlassöffnung (18) zum Aufnehmen des Stomas; und
das zweiteilige Trennschichtsystem (20, 22), das an der Hautbarriere befestigt ist;
wobei das zweiteilige Trennschichtsystem dazu konfiguriert ist, einem Benutzer zu ermöglichen, den Klebstoff auf der peristomalen Haut freizulegen, während die Hautbarriere mit dem Stoma ausgerichtet ist und dieses umgibt.

3. Klebevorrichtung nach Anspruch 1 oder 2, wobei das zweiteilige Trennschichtsystem (20, 22) derart konfiguriert ist, dass die erste Trennschicht (20) etwa eine Hälfte des Klebstoffs und die zweite Trennschicht (22) die andere Hälfte des Klebstoffs bedeckt, wobei eine Teilung zwischen der ersten und der zweiten Trennschicht in der Nähe einer Mitte des Klebstoffs definiert ist.

4. Klebevorrichtung nach Anspruch 2 oder 3, wobei das zweiteilige Trennschichtsystem (20, 22) eine mittige Öffnung (34, 52) beinhaltet, die mit der Einlassöffnung ausgerichtet ist.

5. Klebevorrichtung nach einem der Ansprüche 1-4, wobei mindestens eine der ersten und der zweiten Trennschicht (20, 22) dazu konfiguriert ist, sich über einen Umfang des Klebstoffs hinaus zu erstrecken und/oder wobei die zweite Trennschicht (22) die erste Trennschicht (20) in der Nähe einer Mitte des Klebstoffs überlappt.

6. Klebevorrichtung nach einem der Ansprüche 1-5, wobei die erste Trennschicht (20) den ersten Greifabschnitt (26), einen ersten Mittelabschnitt (28) und den ersten Kontaktabschnitt (30) beinhaltet, wobei der erste Greifabschnitt an einer ersten Falte (36) auf den ersten Mittelabschnitt gefaltet wird und der erste Mittelabschnitt an einer zweiten Falte (38) auf den ersten Kontaktabschnitt gefaltet wird, wobei die erste Trennschicht derart konfiguriert ist, dass sich der erste Greifabschnitt über einen Umfang der Klebevorrichtung hinaus erstreckt, wenn der erste Greifabschnitt vom ersten Mittelabschnitt weggeklappt wird.

7. Klebevorrichtung nach Anspruch 6, wobei die erste Trennschicht (20) dazu konfiguriert ist, durch eine Zugbewegung eines Benutzers vom Klebstoff entfernt zu werden, wobei das mindestens eine Fingerloch (32) dazu konfiguriert ist, einen Finger des Benutzers aufzunehmen, um ein Greifen des ersten Greifabschnitts (26) und ein Abziehen des ersten Kontaktabschnitts (30) in Richtung eines Außenumfangs der Klebevorrichtung zu erleichtern.

8. Klebevorrichtung nach Anspruche 6 oder 7, wobei die zweite Trennschicht (22) als ein Spiegelbild der ersten Trennschicht (20) konfiguriert ist und einen zweiten Greifabschnitt (44), einen zweiten Mittelabschnitt (46) und einen zweiten Kontaktabschnitt (48) beinhaltet, wobei der zweite Greifabschnitt an einer dritten Falte (54) auf den zweiten Mittelabschnitt gefaltet wird und der zweite Mittelabschnitt an einer vierten Falte (56) auf den zweiten Kontaktabschnitt gefaltet wird, wobei die zweite Trennschicht derart konfiguriert ist, dass sich der zweite Greifabschnitt (44) über einen Umfang der Klebevorrichtung hinaus erstreckt, wenn der zweite Greifabschnitt vom zweiten Mittelabschnitt weggeklappt wird.

9. Klebevorrichtung nach Anspruch 8, wobei die zweite Trennschicht (22) dazu konfiguriert ist, durch eine Zugbewegung eines Benutzers vom Klebstoff entfernt zu werden, wobei der zweite Greifabschnitt (44) mindestens ein Fingerloch (50) beinhaltet, das dazu konfiguriert ist, einen Finger des Benutzers aufzunehmen, um ein Greifen des zweiten Greifabschnitts und ein Abziehen des zweiten Kontaktabschnitts (48) in Richtung eines Außenumfangs der Klebevorrichtung zu erleichtern.

10. Klebevorrichtung nach einem der Ansprüche 8-9, wobei das zweiteilige Trennschichtsystem (20, 22) einen Streifen Klebeband (70) beinhaltet, der dazu konfiguriert ist, den ersten und den zweiten Greifabschnitt (26, 44) zusammenzuhalten.

11. Klebevorrichtung nach einem der Ansprüche 6-10, wobei eine dem Klebstoff zugewandte Oberfläche des ersten Kontaktabschnitts (30) und eine dem Klebstoff zugewandte Oberfläche des zweiten Kontaktabschnitts (48) mit einer Trennbeschichtung beschichtet sind, die zur trennbaren Befestigung des ersten und des zweiten Kontaktabschnitts (30, 48) am Klebstoff konfiguriert ist.

12. Klebevorrichtung nach einem der Ansprüche 6-11, wobei mindestens ein Abschnitt des ersten und des zweiten Greifabschnitts (26, 44) und/oder mindestens ein Abschnitt des ersten und des zweiten Mittelabschnitts (28, 46) als eine beschreibbare Oberfläche konfiguriert ist, um einem Benutzer zu ermöglichen, auf der beschreibbaren Oberfläche unter Verwendung eines Marker oder eines Stifts zu schreiben.

13. Klebevorrichtung nach einem der Ansprüche 1-12, wobei die erste und die zweite Trennschicht (20, 22) ein verstärktes Material umfassen, um ein Reißen während des Entfernens zu verhindern, und/oder die Klebevorrichtung ferner einen Klemmmechanismus umfasst, der dazu konfiguriert ist, ein Ergreifen und Entfernen der ersten und der zweiten Trennschicht (20, 22) zu erleichtern.

14. Klebevorrichtung nach einem der Ansprüche 2 bis 13, wobei die Hautbarriere (16) eine konvexe Barriere oder eine konkave Hautbarriere ist.

15. Klebevorrichtung nach Anspruch 1, wobei die Klebevorrichtung eine medizinische Klebevorrichtung zum Befestigen eines Saugschlauchs, eines Drainageschlauchs, eines vertikalen Schlauchs, eines Ernährungsschlauchs, eines horizontalen Schlauchs oder eines oralen Endotrachealtubus an einem Benutzer ist.

## Revendications

1. Dispositif adhésif pour une fixation sur la peau comprenant :
un adhésif pour une fixation sur la peau ; et
un système de doublure détachable en deux parties (20, 22) pour une protection de l'adhésif,
dans lequel le système de doublure détachable en deux parties comporte une première doublure détachable (20) et une seconde doublure détachable (22), dans lequel la première doublure détachable comporte une première portion de contact (30) configurée pour être fixée à l'adhésif et une première portion de préhension (26), **caractérisé en ce que** la première portion de préhension comporte au moins un trou pour un doigt (32) configuré pour faciliter le retrait de la première doublure détachable par un utilisateur.

2. Dispositif adhésif selon la revendication 1, dans lequel le dispositif adhésif est un ensemble barrière de stomie (10) pour la fixation d'un appareil de stomie sur une peau péristomiale entourant un stoma comprenant :
une barrière cutanée (16) comprenant l'adhésif ;
une ouverture d'entrée (18) délimitée dans la barrière cutanée pour recevoir le stoma ; et
le système de doublure détachable en deux parties (20, 22) fixé à la barrière cutanée ;
dans lequel le système de doublure détachable en deux parties est configuré pour permettre à un utilisateur d'exposer l'adhésif à la peau péristomiale tandis que la barrière cutanée est alignée avec le stoma et entoure celui-ci.

3. Dispositif adhésif selon la revendication 1 ou 2, dans lequel le système de doublure détachable en deux parties (20, 22) est configuré de telle sorte que la première doublure détachable (20) recouvre environ une moitié de l'adhésif et la seconde doublure détachable (22) recouvre l'autre moitié de l'adhésif, dans lequel une séparation entre les première et seconde doublures détachables est définie à proximité d'un centre de l'adhésif.

4. Dispositif adhésif selon la revendication 2 ou 3, dans lequel le système de doublure détachable en deux parties (20, 22) comporte une ouverture centrale (34, 52) alignée avec l'ouverture d'entrée.

5. Dispositif adhésif selon l'une quelconque des revendications 1 à 4, dans lequel au moins l'une des première et seconde doublures détachables (20, 22) est configurée pour s'étendre au-delà d'une périphérie de l'adhésif et/ou dans lequel la seconde doublure détachable (22) chevauche la première doublure détachable (20) à proximité d'un centre de l'adhésif.

6. Dispositif adhésif selon l'une quelconque des revendications 1 à 5, dans lequel la première doublure détachable (20) comporte la première portion de préhension (26), une première portion médiane (28) et la première portion de contact (30), dans lequel la première portion de préhension se replie sur la première portion médiane au niveau d'un premier pli (36) et la première portion médiane se replie sur la première portion de contact au niveau d'un deuxième pli (38), dans lequel la première doublure détachable est configurée de telle sorte que la première portion de préhension s'étende au-delà d'une périphérie du dispositif adhésif lorsque la première portion de préhension est dépliée de la première portion médiane.

7. Dispositif adhésif selon la revendication 6, dans lequel la première doublure détachable (20) est configurée pour être retirée de l'adhésif par un mouvement de traction de l'utilisateur, dans lequel l'au moins un trou pour un doigt (32) est configuré pour recevoir un doigt de l'utilisateur afin de faciliter la préhension de la première portion de préhension (26) et la traction de la première portion de contact (30) vers une périphérie extérieure du dispositif adhésif.

8. Dispositif adhésif selon la revendication 6 ou 7, dans lequel la seconde doublure détachable (22) est configurée comme une image miroir de la première doublure détachable (20) et comporte une seconde portion de préhension (44), une seconde portion médiane (46) et une seconde portion de contact (48), dans lequel la seconde portion de préhension se replie sur la seconde portion médiane au niveau d'un troisième pli (54) et la seconde portion médiane se replie sur la seconde portion de contact au niveau d'un quatrième pli (56), dans lequel la seconde doublure détachable est configurée de telle sorte que la seconde portion de préhension (44) s'étende au-delà d'une périphérie du dispositif adhésif lorsque la seconde portion de préhension est dépliée de la seconde portion médiane.

9. Dispositif adhésif selon la revendication 8, dans lequel la seconde doublure détachable (22) est configurée pour être retirée de l'adhésif par un mouvement de traction de l'utilisateur, dans lequel la seconde portion de préhension (44) comporte au moins un trou pour un doigt (50) configuré pour recevoir un doigt de l'utilisateur afin de faciliter la préhension de la seconde portion de préhension et la traction de la seconde portion de contact (48) vers une périphérie extérieure du dispositif adhésif.

10. Dispositif adhésif selon l'une quelconque des revendications 8 à 9, dans lequel le système de doublure détachable en deux parties (20, 22) comporte une bande de ruban adhésif (70) configurée pour maintenir ensemble les première et seconde portions de préhension (26, 44).

11. Dispositif adhésif selon l'une quelconque des revendications 6 à 10, dans lequel une surface orientée vers l'adhésif de la première portion de contact (30) et une surface orientée vers l'adhésif de la seconde portion de contact (48) sont revêtues d'un revêtement détachable configuré pour la fixation détachable des première et seconde portions de contact (30, 48) à l'adhésif.

12. Dispositif adhésif selon l'une quelconque des revendications 6 à 11, dans lequel au moins une portion parmi les première et seconde portions de préhension (26, 44) et/ou au moins une portion parmi les première et seconde portions médianes (28, 46) est configurée comme une surface inscriptible pour permettre à un utilisateur d'écrire sur la surface inscriptible à l'aide d'un marqueur ou d'un stylo.

13. Dispositif adhésif selon l'une quelconque des revendications 1 à 12, dans lequel les première et seconde doublures détachables (20, 22) comprennent un matériau renforcé pour éviter toute déchirure lors du retrait, et/ou le dispositif adhésif comprenant en outre un mécanisme de serrage configuré pour faciliter la saisie et le retrait des première et seconde doublures détachables (20, 22).

14. Dispositif adhésif selon l'une quelconque des revendications 2 à 13, dans lequel la barrière cutanée (16) est une barrière convexe ou une barrière cutanée concave.

15. Dispositif adhésif selon la revendication 1, dans lequel le dispositif adhésif est un dispositif adhésif médical pour la fixation d'un tube d'aspiration, d'un tube de drain, d'un tube vertical, d'un tube d'alimentation, d'un tube horizontal ou d'un tube endotrachéal oral à un utilisateur.
